(19) Europäisches Patentamt
European Patent Office
Office européen des brevets

(11) Veröffentlichungsnummer: **0 091 508**
**B1**

(12) # EUROPÄISCHE PATENTSCHRIFT

(45) Veröffentlichungstag der Patentschrift: **23.07.86**

(51) Int. Cl.[4]: **C 01 B 35/12,** B 01 J 29/04, C 07 C 1/20

(21) Anmeldenummer: **82109667.4**

(22) Anmeldetag: **20.10.82**

(54) **Borosilikatzeolithe-ZBH und Verfahren zur Herstellung von kristallinen Borosilikatzeolithen (ZBH) und deren Verwendung als Katalysator.**

(30) Priorität: **30.10.81 DE 3143045**

(43) Veröffentlichungstag der Anmeldung: **19.10.83 Patentblatt 83/42**

(45) Bekanntmachung des Hinweises auf die Patenterteilung: **23.07.86 Patentblatt 86/30**

(84) Benannte Vertragsstaaten: **BE DE FR GB NL**

(56) Entgegenhaltungen:
**EP-A-0 007 081**
**EP-A-0 034 727**
**FR-A-2 367 701**
**FR-A-2 429 182**

(73) Patentinhaber: **BASF Aktiengesellschaft, Carl-Bosch- Strasse 38, D-6700 Ludwigshafen (DE)**

(72) Erfinder: **Hoelderich, Wolfgang, Dr., Mannheimer Strasse 18 C, D-6710 Frankenthal (DE)**
Erfinder: **Mross, Wolf Dieter, Dr., Anselm-Feuerbach- Strasse 21, D-6710 Frankenthal (DE)**
Erfinder: **Schwarzmann, Matthias, Dr., Carl- Bosch-Strasse 54, D-6703 Limburgerhof (DE)**

**EP 0 091 508 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Die Erfindung betrifft neue Borosilikatzeolithe-ZBH und ein Verfahren zur Herstellung kristalliner Borosilikatzeolithe-ZBH vom Pentasiltyp in reinem oder wäßrig-alkoholischem Medium ohne Verwendung von Aminen sowie die Verwendung als Katalysatoren. Diese ZBH-Zeolithe katalysieren die Umwandlung von Methanol und/oder Dimethylether zu niederen Olefinen und/oder Aromaten.

Bor-Zeolithe sind bisher in der Natur nicht gefunden worden. Man erhält sie nur auf synthetischem Wege (DE—A—27 46 790). Al-haltige zeolithische Materialien, sowohl natürlicher als auch synthetischer Herkunft, haben sich als katalytisch wirksam erwiesen für verschiedene Arten von Kohlenwasserstoffumwandlung, z.B. in der Technik zum Cracken von Kohlenwasserstoffen, und finden auch als Ionenaustauscher und Molekularsiebe technische Verwendung.

Allgemein besitzen Zeolithe eine hochgeordnete Struktur mit einem starren dreidimensionalen Netzwerk von $SiO_4$- und $MeO_4$-Tetraedern, wobei Me z.B. Al, Fe, Ga und B sein kann. Diese Tetraeder sind durch gemeinsame Sauerstoffatome verbunden. Das Verhältnis der Si- und Me-Atome zu Sauerstoff beträgt 1:2.

Die Eltrovalenz der dreiwertige Elemente Me-enthaltenden Tetraeder ist durch Einschluß von Kationen in den Kristall, z.B. eines Alkali- oder Wasserstoffions ausgeglichen. Ein Kationenaustausch ist möglich. Die Räume zwischen den Tetraedern sind vor der Dehydration durch Trocknen bzw. Calcinieren von Wassermolekeln besetzt.

In den letzten Jahren gewannen kristalline Aluminosilikate mit einem hohen $SiO_2/Al_2O_3$-Verhältnis>11 zunehmendes Interesse. Derartige Zeolithe besitzen Pentasilstruktur und zeichnen sich durch hohe thermische Stabilität und außerordentlich hohe Acidität aus. Die Synthese dieser Zeolithe erfolgt in bekannter Weise aus einer Silicium- und einer Aluminiumkomponente in Gegenwart von voluminösen quaternären organischen Ammonium- bzw. Phosphoniumverbindungen und Alkaliverbindungen als Mineralisierungsmittel. Ein derartiges Verfahren ist z.B. in US—A—3 702 886 beschrieben. Die großen Ammonium- bzw. Phosphoniumionen sollen als Templat für die gewünschte Zeolithstruktur wirken und ermöglichen die Synthese von Aluminium-Zeolithen mit $SiO_2/Al_2O_3$-Verhältnissen z.B. von 100 bis zu 3000.

Bor-Zeolithe mit Pentasil-Struktur wurden bisher nur in Gegenwart von Aminen hergestellt.

In dem EP—A—7081 ist bereits vorgeschlagen kristalline Zeolithe alkalifrei durch hydrothermale Kirstallisation mit Hexamethylendiamin herzustellen. Dabei erhält man aber stickstoffhaltigen bzw. aminhaltige Kristalle, aus denen der basische Stickstoff bzw. die Amine vor manchen Anwendungen der Kristalle, z.B. als Katalysatoren, z.B. durch Abbrennen, entfernt werden muß.

Gegenstand der Erfindung sind neue-Borosilikatzeolithe- ZBH der allgemeinen Formel

$$M_{2/n}O:B_2O_3:x\ SiO_2:y\ H_2O$$

worin M zumindest ein Kation mit einer Wertigkeit n bedeutet, x 10 beträgt und y zwischen 0 und 80 liegt, gemäß folgendem vereinfachten Röntgebeugungsdiagramm:

| Interplanarer Abstand d (Å)*) | | Relative Intensität $I/I_0$ |
|---|---|---|
| 11.0240 | ±0,15 | 93 |
| 9.8992 | | 63 |
| 9.6825 | | 20 |
| 3.8212 | ±0,07 | 100 |
| 3.7908 | | 73 |
| 3.7202 | | 36 |
| 3.6901 | | 49 |
| 3.6154 | | 31 |
| 1.9975 | | 9 |
| 1.9770 | | 10 |

*) 1Å=0,1 nm

Ein weiterer Gegenstand der Erfindung ist ein Verfahren zur Herstellung dieser kristallinen Borosilikatzeolithen-ZBH vom Pentasiltyp durch hydrothermale Kristallisation von Siliciumdioxid und

Borsäure in Gegenwart von Alkalisalzen, bei dem man die Kristallisation bei Temperaturen von 80 bis 150°C in alkoholischer oder wäßrig-alkoholischer Mischung ausführt.

Eine bevorzugte Ausführungsform zur Herstellung dieser aminfreien Borosilikatzeolithe-ZBH besteht darin, daß man eine Reaktionsmischung aus $SiO_2$, z.B. eine käufliche pyrogene Kieselsäure, $H_3BO_3$ und NaOH in einer Alkohol/Wasser-Mischung von 50:50 Gew.% 5 bis 7 Tage bei 110 bis 130°C unter autogenem Druck in einem Rührautoklaven umsetzt. Das zunächst erhaltene Reaktionsprodukt kann vor der Calcinierung noch Alkohol enthalten anstelle der in den intrakristallinen Poren enthaltenen $H_2O$-Molekeln. Alle Reste organischer Verbindungen werden durch Trocknen und Calcinieren entfernt, so daß nur noch die freien Alkaliionen im Bor-Zeolith enthalten sind.

Das Molverhältnis von $SiO_2$ zu $B_2O_3$ in der Reaktionsmischung wird zwischen 3 und 300, vorzugsweise bei 10 bis 50 eingestellt.

Der Anteil an Alkalihydroxid liegt bei 1 bis 3 Mol pro Äquivalent $B_2O_3$, bevorzugt bei 1,5 Mol.

Es ist zweckmäßig, Impfkristalle zur Reaktionsführung und -beschleunigung zuzusetzen.

Als Lösungsmittel kann man allgemein ein-, zwei- oder mehr-wertige, primäre, sekundäre oder tertiäre Alkohole z.B. $CH_3OH$, $CH_3CH_2OH$, $(CH_3)_2CH—OH$, Butandiol, Hexandiol einsetzen, vorzugsweise verwendet man 1,4-Butandiol und/oder 1,6-Hexandiol.

Die molare Zusammensetzung der Reaktionsmischung wird wie folgt eingestellt:

$SiO_2/B_2O_3=3—300$, vorzugsweise 10—50

$M_2O/B_2O_3=>1$, vorzugsweise 1,3—2

$ROH/B_2O_3=18—390$

wobei M=Alkali, insbesondere Na und ROH einen Alkohol bedeutet. Die Konzentration der Ether in $H_2O$ kann zwischen 10 und 100 Gew.% liegen, bevorzugt 50 Gew.%.

Das erfindungsgemäße Verfahren wird bei Reaktionstemperaturen im Bereich von 80 bis 150°C, vorzugsweise zwischen 110 und 130°C und vorteilhafterweise unter dem Eigendruck in einem Rührautoklaven aus Edelstahl während 3 bis 7 Tagen durchgeführt.

Nach der Reaktion wird das Reaktionsprodukt zweckmäßigerweise abfiltriert, mit Wasser gut ausgewaschen und bei 110°C/16 h getrocknet. Anschließend wird das Produkt bei 550°C/20 h calciniert, um den noch eingeschlossenen Alkohol herauszubrennen und den Bor-Zeolith zu dehydratisieren. Die Überführung der Alkaliform des Zeolithen in die katalytisch aktive H-Form kann nach bekannten Austauschverfahren, z.B. mit Ammoniumsalzen, erfolgen.

Die allgemeine Formel der hier hergestellten Borosilikatzeolithe-ZBH lautet:

$$M_{2}/_{n}O:B_2O_3:x\ SiO_2:y\ H_2O$$

worin M zumindest ein Kation mit einer Wertigkeit n bedeutet, x 10 beträgt und y zwischen 0 und 80 liegt.

Die erfindungsgemäß im Alkohol hergestellten Borisilikatzeolithe-ZBH zeigen folgende Röntgenbeugungslinien:

TABELLE 1

| Interplanarer Abstand d (Å) | | Relative Intensität $I/I_o$ |
|---|---|---|
| 11.1046 | ±0,15 | 81 |
| 10.0048 | | 59 |
| 9.7090 | | 21 |
| 6.3280 | | 16 |
| 5.9655 | ±0,1 | 18 |
| 5.6724 | | 11 |
| 5.5438 | | 16 |
| 4.5933 | | 9 |
| 4.3353 | | 12 |
| 4.2395 | | 12 |
| 3.9826 | ±0,07 | 7 |
| 3.8332 | | 100 |
| 3.7980 | | 70 |
| 3.6984 | | 50 |
| 3.6241 | | 27 |
| 3.4641 | | 7 |
| 3.4230 | | 9 |
| 3.3773 | | 6 |
| 3.3283 | | 9 |
| 3.2955 | | 11 |
| 3.0302 | | 12 |
| 2.9719 | | 12 |
| 2.9240 | | 9 |
| 2.7191 | | 4 |
| 2.5905 | | 4 |
| 2.4741 | | 5 |
| 2.4021 | | 4 |
| 2.3841 | | 5 |
| 2.0957 | | 2 |
| 2.0545 | | 2 |
| 1.9998 | | 10 |

TABELLE 1: Fortsetzung

| Interplanarer Abstand d (Å) | Relative Intensität $I/I_o$ |
|---|---|
| 1.9791 | 9 |
| 1.9393 | 3 |
| 1.9029 | 3 |
| 1.8605 | 3 |
| 1.7565 | 2 |
| 1,7512 | 2 |
| 1,7464 | 2 |
| 1,7405 | 3 |
| 1,6600 | 4 |
| 1,6509 | 3 |
| 1,6471 | 2 |
| 1,6015 | 2 |
| 1,5539 | 2 |
| 1.4877 | 1 |
| 1.4535 | 3 |
| 1.4364 | 3 |
| 1.4125 | 0 |
| 1.3859 | 2 |
| 1.3753 | 2 |

Das Röntgenbeugungsdiagramm wird mit einem automatischen Pulverdiffraktometer APD-10 hergestellt. Es wird Kupferstrahlung zusammen mit einem Graphitmonochromator verwendet.

Das für die in Alkohol hergestellten Borosilikatzeolithe-ZBH typische Röntgenbeugungsmuster weist diese als Mitglieder der Pentasilfamilie aus.

In den erfindungsgemäßen Borosilikatzeolithen-ZBH ist das Bor in die tetraedrischen Gitterplätze des Kristallgerüstes eingebaut. Der Einbau von Bor in das Kristallgerüst kommt in der Verschiebung der Röntgenbeugungslinien nach kleineren d-Werten im Vergleich zu entsprechenden Aluminosilikatzeolithen zum Ausdruck (Tabelle 2); die Bindungslänge einer B-O-Bindung ist erheblich kürzer als der Al-O-Abstand. Der Einbau des Bors in das Kristallgerüst bewirkt daher eine Kontraktion der Elementarzelle, d.h. eine Verschiebung der d-Abstände nach kleineren Werten im Vergleich zu entsprechenden Aluminosilikatzeolithen bzw. im Vergleich zu den d-Abständen des Silicalith.

Ein Vergleich der Netzebenenabstände der hier beschriebenen Borzeolithe mit denen des ZSM-5 zeigt, daß die d-Werte (Å) beim Borzeolith kleiner sind als beim Aluminosilikat ZSM-5 (Tabelle 2).

TABELLE 2

| ZBH d (Å) | ZBH I/I$_o$ | ZSM-5[1] d (A) | ZSM-5[1] I | AMS—1 B[2] d (X) | AMS—1 B[2] Relative Intensität |
|---|---|---|---|---|---|
| 11.0096 | 100 | 11.36 | s | 11,3 ±0,2 | 38 |
| 9.8917 | 64 | 10.20 | ms | 10,1 ±0,2 | 30 |
| 9.6372 | 21 | 9.90 | — | 6,01±0,07 | 14 |
| 6.6346 | 8 | 9.14 | vw | 4,35±0,05 | 11 |
| 6.2916 | 13 | 7.54 | w | 4,26±0,05 | 14 |
| 5.9391 | 17 | 7.17 | w | 3,84±0,05 | 100 |
| 5.6475 | 11 | 6.79 | vw | 3,72±0,05 | 52 |
| 5.5337 | 12 | 6.06 | w | 3,65±0,05 | 31 |
| 5.3245 | 5 | 5.77 | w | 3,44±0,05 | 14 |
| 5.0647 | 4 | 5.63 | w | 3,33±0,05 | 16 |
| 4.9334 | 9 | 5.42 | vw | 3,04±0,02 | 22 |
| 4.5659 | 7 | 5.19 | vw | 2,97±0,02 | 22 |
| 4.3231 | 10 | 5.05 | w | 2,48±0,02 | 11 |
| 4.2316 | 10 | 4.65 | w | 1,99±0,02 | 20 |
| 4.0597 | 5 | 4.40 | w | 1,66±0,02 | 12 |
| 3.9836 | 5 | 4.30 | w | | |
| 3.8268 | 77 | 4.12 | vw | | |
| 3.7899 | 50 | | | | |
| 3.6918 | 39 | 4.04 | vw | | |
| 3.6234 | 22 | 3.84 | vs | | |
| 3.4590 | 6 | 3.74 | vs | | |
| 3.4442 | 5 | 3.62 | s | | |
| 3.4182 | 8 | 3.50 | w | | |
| 3.3841 | 5 | 3.46 | w | | |
| 3.3307 | 7 | 3.33 | w | | |
| 3.2879 | 9 | 3.27 | vw | | |
| 3.2343 | 4 | 3.07 | w | | |
| 3.1192 | 2 | 3.00 | m | | |
| 3.0233 | 7 | | | | |
| 2.9703 | 14 | | | | |

TABELLE 2: Fortsetzung

| ZBH d (Å) | ZBH $I/I_o$ | ZSM-5[1] d (A) | ZSM-5[1] I | AMS—1 B[2] d (X) | AMS—1 B[2] Relative Intensität |
|---|---|---|---|---|---|
| 2.9464 | 8 | | | | |
| 2.8525 | 2 | | | | |
| 2.7205 | 3 | | | | |
| 2.5987 | 3 | | | | |
| 2.5891 | 3 | | | | |
| 2.5035 | 2 | | | | |
| 2.4722 | 4 | | | | |
| 2.4033 | 3 | | | | |
| 2.3837 | 3 | | | | |
| 2.1659 | 1 | | | | |
| 2.0834 | 1 | | | | |
| 2.0703 | 1 | | | | |
| 2.0574 | 1 | | | | |
| 2.0012 | 8 | | | | |
| 1.9802 | 8 | | | | |
| 1.9425 | 2 | | | | |
| 1.9385 | 2 | | | | |
| 1.9067 | 2 | | | | |
| 1.8662 | 3 | | | | |
| 1.7604 | 2 | | | | |
| 1.7551 | 2 | | | | |
| 1.7407 | 2 | | | | |
| 1.7016 | 1 | | | | |
| 1.6607 | 3 | | | | |
| 1.6204 | 1 | | | | |
| 1.5965 | 2 | | | | |
| 1.5541 | 1 | | | | |
| 1.4779 | 1 | | | | |
| 1.4569 | 3 | | | | |
| 1.4427 | 2 | | | | |

TABELLE 2: Fortsetzung

| ZBH | | ZSM-5[1] | | AMS—1 B[2] | |
|---|---|---|---|---|---|
| d (Å) | $I/I_o$ | d (A) | I | d (X) | Relative Intensität |
| 1.4386 | 2 | | | | |
| 1.4036 | 2 | | | | |
| 1.4020 | 1 | | | | |
| 1.3892 | 2 | | | | |
| 1.3856 | 2 | | | | |
| 1.3838 | 1 | | | | |
| 1.3807 | 2 | | | | |
| 1.3752 | 1 | | | | |
| 1.3582 | 1 | | | | |
| 1.3557 | 1 | | | | |

[1]Zeolithe, Molecular Sieves, Donald W. Breck,
[2]DE—A—27 46 790, Standard Oil Co.

Die hier beschriebenen kristallinen Borosilikatzeolithe-ZBH unterscheiden sich in ihren interplanaren Abständen (d-Werte in Å) deutlich von den Werten des Borozeolithen AMS—1 B der Standard Oil Co. (Tabelle 2).

Auch ein Vergleich der Intensitäten der Röntgenbeugungslinien zeigt deutliche Unterschiede auf.

Die Unterschiede sowohl in den d-Werten als auch in den Intensitäten weisen die ZBH-Borisilikate für den Experten als neuartige Zeolithe aus.

Die in Alkoholen hergestellten Borosilikatzeolith-ZBH gehören zwar im Strukturtyp zur Pentasilfamilie, zeigen jedoch gegenüber den anderen Mitgliedern dieses Strukturtyps deutlich verschiedene Röntgenbeugungslinien und damit andere Gitterabstände.

Die nach Austausch in H-Form erhaltenen Zeolithe können mit Matrixmaterial verstrangt und als Katalysator für Kohlenwasserstoffumwandlungen wie Isomerisierungen, Cracken und Umwandlung von Alkoholen und/oder Ethern in Olefine, Gasoline und Aromaten eingesetzt werden.

Die in den Beispielen angegebenen Analysenwerte beziehen sich auf Trockenbasis. Die Substanzen werden vor der chemischen Analyse bei 550°/20 h calciniert. Die Differenz zu 100% ergibt sich durch adsorbiertes Wasser.

Beispiele 1 bis 5

In 1000 g Alkohol/$H_2O$-Gemisch (50:50) werden bei ca. 40°C 80 g Aerosil eingerührt. 15,2 g $H_3BO_3$ werden in 200 g Alkohol/$H_2O$-Gemisch gelöst. Beide Lösungen werden zusammengegeben. Danach werden 14,7 g NaOH und eventuell Impfkristalle zugesetzt. Die homogen gerührte Mischung wird in einem Rührautoklaven 5 Tage bei Temperaturen zwischen 90 und 150°C unter autogenem Durck umgesetzt. Nach Abkühlen des Reaktionsgemisches wird das kristalline Produkt abfiltriert, mit 10 l Wasser ausgewaschen, 16 h bei 110°C getrocknet und 20 h bei 500°C calciniert.

Die Beispiele 1 bis 5 sind in der Tabelle 3 zusammengefaßt. Die synthetischen Borzeolithe wurden ohne Verunreinigungen erhalten.

Die interplanaren Netzebenenabstände (d-Werte in Å) und die Intensitäten der Röntgenbeugungslinien sind für die in den einzelnen Beispielen angegebenen Borosilikatzeolithe in Tabelle 4 zusammengefaßt.

Beispiel 6

Der in Beispiel 4 in 1,4-Butandiol hergestellte Borosilikatzeolith-ZBH wird mit Böhmit im Verhältnis 60:40 verstrangt. Das Überführen der Na-Form in die katalytisch aktive H-Form des Zeolithen erfolgt durch Austausch mit $NH_4Cl$ (20 %ige $NH_4Cl$-Lösung) im Verhältnis 1:15 in 2 h bei 80°C.

80 %iges Methanol wird bei 450°C bei einer Belastung von WHSV=7,8 h-1 zu 100% umgesetzt. Die Ausbeuten, bezogen auf eingesetztes $CH_2$, sind

| | |
|---|---|
| $CH_4$ | 1,9% |
| $C_2H_6$ | 0,1% |
| $C_3H_8$ | 1,13% |
| $C_5$-KW | 28,9% |
| $C_2H_4$ | 5,7% |
| $C_3H_6$ | 23,5% |
| $X_4$-KW | 17,8% |

Reste sind z.B. CO und $H_2$

TABELLE 3

| Beispiel | Alkohol | eingesetztes $SiO_2/B_2O_3$ | gef. $SiO_2$ % | gef. $B_2O_3$ % | gef. $SiO_2/B_2O_3$ % | gef. Na % |
|---|---|---|---|---|---|---|
| 1 | Methanol | 11 | 87,1 | 4,34 | 23,4 | 3,8 |
| 2 | Ethanol | 11 | 85,4 | 4,18 | 23,8 | 3,8 |
| 3 | n-Butanol | 11 | 91,1 | 2,2 | 48,3 | 2,3 |
| 4 | 1,4-Butandiol | 11 | 82,1 | 8,37 | 11,4 | 5,88 |
| 5 | 1,6-Hexandiol | 11 | 92,4 | 2,97 | 36,3 | 2,3 |

TABELLE 4

| Beispiel 1 d(Å) | $I/I_o$ | Beispiel 2 d(Å) | $I/I_o$ | Beispiel 3 d(Å) | $I/I_o$ | Beispiel 4 d(Å) | $I/I_o$ | Beispiel 5 d(Å) | $I/I_o$ |
|---|---|---|---|---|---|---|---|---|---|
| 11.0240 | 93 | 11.1046 | 81 | 11.0945 | 100 | 11.0958 | 99 | 11.0096 | 100 |
| 9.8992 | 63 | 10.0048 | 59 | 9.9763 | 76 | 9.9782 | 78 | 9.8917 | 64 |
| 9.6825 | 20 | 9.7090 | 21 | 9.6646 | 25 | 9.6773 | 27 | 9.6372 | 21 |
| 6.3059 | 15 | 6.3280 | 16 | 6.6829 | 10 | 6.6710 | 10 | 6.6346 | 8 |
| 5.9305 | 19 | 5.9655 | 13 | 6.3288 | 16 | 6.3227 | 18 | 6.2916 | 13 |
| 5.6458 | 12 | 5.6724 | 11 | 5.9723 | 20 | 5.9632 | 23 | 5.9391 | 17 |
| 5.5250 | 16 | 5.5438 | 16 | 5.6918 | 12 | 5.6774 | 13 | 5.6475 | 11 |
| 5.3212 | 6 | 4.5933 | 9 | 5.5543 | 16 | 5.5463 | 19 | 5.5337 | 12 |
| 5.1037 | 6 | 4.3353 | 12 | 5.0099 | 8 | 5.1316 | 6 | 5.3245 | 5 |
| 4.9471 | 10 | 4.2395 | 12 | 4.9775 | 9 | 4.9975 | 10 | 5.0647 | 4 |
| 4.5689 | 8 | 3.9826 | 7 | 4.5995 | 8 | 4.9527 | 11 | 4.9334 | 9 |
| 4.4021 | 6 | 3.8332 | 100 | 4.3508 | 14 | 4.6051 | 8 | 4.5659 | 7 |
| 4.3313 | 13 | 3.7980 | 70 | 4.3337 | 11 | 4.3469 | 12 | 4.3231 | 10 |
| 4.2253 | 12 | 3.6984 | 50 | 4.2457 | 12 | 4.2384 | 14 | 4.2316 | 10 |
| 3.9688 | 6 | 3.6241 | 27 | 3.8423 | 89 | 3.8337 | 100 | 4.0597 | 5 |
| 3.8212 | 100 | | | | | | | | |
| 3.7908 | 73 | 3.4641 | 7 | 3.8090 | 53 | 3.8024 | 73 | 3.9836 | 5 |
| 3.7202 | 36 | 3.4230 | 9 | 3,7067 | 47 | 3.7249 | 39 | 3.8268 | 77 |
| 3.6901 | 49 | 3.3773 | 6 | 3.6385 | 19 | 3.7038 | 50 | 3.7899 | 50 |
| 3.6637 | 13 | 3.3283 | 9 | 3.4298 | 26 | 3.6266 | 28 | 3.6918 | 39 |
| 3.6154 | 31 | 3.2955 | 11 | 3.4176 | 25 | 3.4223 | 14 | 3.6234 | 22 |
| 3.4528 | 7 | 3.0302 | 12 | 3.4003 | 25 | 3.3889 | 10 | 3.4590 | 6 |
| 4.4177 | 10 | 2.9719 | 12 | 3.3878 | 25 | 3.3418 | 11 | 3.4442 | 5 |
| 3.3207 | 10 | 2.9240 | 9 | 3.3516 | 25 | 3.2954 | 13 | 3.4182 | 8 |
| 3.2897 | 11 | 2.7191 | 4 | 3.0389 | 8 | 3.0361 | 10 | 3.3841 | 5 |
| 3.0266 | 11 | 2.5905 | 4 | 2.9785 | 17 | 2.9747 | 16 | 3.3307 | 7 |
| 2.9677 | 14 | 2.4741 | 5 | 2.9490 | 8 | 2.9593 | 11 | 3.2879 | 9 |
| 2.9525 | 11 | 2.4021 | 4 | 2.7325 | 5 | 2.7202 | 6 | 3.2343 | 4 |
| 2.9198 | 8 | 2.3841 | 5 | 2.3932 | 4 | 2.6015 | 18 | 3.1192 | 2 |
| 2.7129 | 4 | 2.0957 | 2 | 2.3881 | 4 | 2.5026 | 5 | 3.0233 | 7 |

TABELLE 4: Fortsetzung

| Beispiel 1 d(Å) | $I/I_o$ | Beispiel 2 d(Å) | $I/I_o$ | Beispiel 3 d(Å) | $I/I_o$ | Beispiel 4 d(Å) | $I/I_o$ | Beispiel 5 d(Å) | $I/I_o$ |
|---|---|---|---|---|---|---|---|---|---|
| 2.5867 | 4 | 2.0545 | 2 | 2.0062 | 10 | 2.4767 | 5 | 2.9703 | 14 |
| 2.4681 | 5 | 1.9998 | 10 | 2.0029 | 8 | 2.4061 | 4 | 2.9464 | 8 |
| 2.3070 | 3 | 1.9791 | 9 | 1.9841 | 9 | 2.3862 | 5 | 2.8525 | 2 |
| 2.0550 | 2 | 1.9393 | 3 | 1.8351 | 4 | 2.3141 | 3 | 2.7205 | 3 |
| 1.9975 | 9 | 1.9029 | 3 | 1.6643 | 4 | 2.0033 | 10 | 2.5987 | 3 |
| 1.9770 | 10 | 1.8605 | 3 | 1.6619 | 3 | 1.9817 | 10 | 2.5891 | 3 |
| 1.8997 | 3 | 1.7565 | 2 | 1.4583 | 4 | 1.4951 | 5 | 2.5035 | 2 |
| 1.8571 | 4 | 1.7512 | 2 | | | 1.9034 | 4 | 2.4722 | 4 |
| 1.7568 | 2 | 1.7464 | 2 | | | 1.8651 | 3 | 2.4033 | 3 |
| 1.7519 | 2 | 1.7405 | 3 | | | 1.7614 | 3 | 2.3837 | 3 |
| 1.7432 | 3 | 1.6600 | 4 | | | 1.6640 | 4 | 2.1659 | 1 |
| 1.6553 | 4 | 1.6509 | 3 | | | 1.6544 | 3 | 2.0834 | 1 |
| 1.6456 | 3 | 1.6471 | 2 | | | 1.4561 | 4 | 2.0703 | 1 |
| 1.6028 | 2 | 1.6015 | 2 | | | 1.4025 | 3 | 2.0574 | 1 |
| 1.4536 | 3 | 1.5539 | 2 | | | 1.3912 | 4 | 2.0012 | 8 |
| 1.4353 | 3 | 1.4877 | 1 | | | 1.3874 | 3 | 1.9802 | 8 |
| 1.4113 | 2 | 1.4535 | 3 | | | 1.3831 | 3 | 1.9425 | 2 |
| 1.3849 | 2 | 1.4364 | 3 | | | 1.3576 | 2 | 1.9385 | 2 |
| 1.3565 | 0 | 1.4125 | 0 | | | | | 1.9067 | 2 |
| 1.3541 | 2 | 1.3859 | 2 | | | | | 1.8662 | 3 |
| | | 1.3753 | 2 | | | | | 1.7604 | 2 |
| | | | | | | | | 1.7551 | 2 |
| | | | | | | | | 1.7407 | 2 |
| | | | | | | | | 1.7016 | 1 |
| | | | | | | | | 1.6607 | 3 |
| | | | | | | | | 1.5965 | 2 |
| | | | | | | | | 1.5541 | 1 |
| | | | | | | | | 1.4779 | 1 |

**Patentansprüche**

1. Borosilikatzeolithe-ZBH der allgemeinen Formel

$$M_{2/n}O:B_2O_3:x\ SiO_2:y\ H_2O$$

worin M zumindest ein Kation mit einer Wertigkeit n bedeutet, x 10 beträgt und y zwischen 0 und 80 liegt, gemäß folgenden vereinfachtem Röntgenbeugungsdiagramm:

| Interplanarer Abstand d (Å)*) | Relative Intensität $I/I_0$ |
|---|---|
| 11.0240 ±0,15 | 93 |
| 9.8992 | 63 |
| 9.6825 | 20 |
| 3.8212 ±0,07 | 100 |
| 3.7908 | 73 |
| 3.7202 | 36 |
| 3.6901 | 49 |
| 3.6154 | 31 |
| 1.9975 | 9 |
| 1.9770 | 10 |

*) 1Å=0,1 nm

2. Verfahren zur Herstellung von kristallinen Borosilikatzeolithen-ZBH vom Pentasiltyp gemäß Anspruch 1 durch hydrothermale Kristallisation von Siliciumdioxid und Borsäure in Gegenwart von Alkalisalzen, dadurch gekennzeichnet, daß man die Kristallisation bei Temperaturen von 80 bis 150°C in alkoholischer oder wäßrig-alkoholischer Mischung ausführt.

3. Verfahren nach Anspruch 2, dadurch gekennzeichnet, daß man als Lösungsmittel für die Kristallisation 1,6-Hexandiol und/oder 1,4-Butandiol verwendet.

4. Verfahren nach Anspruch 2 und 3, dadurch gekennzeichnet, daß die Mischung Alkalisalze in Mengen von 1 bis 3 Mol pro Mol $B_2O_3$ enthält.

5. Verfahren nach Anspruch 2 bis 4, dadurch gekennzeichnet, daß man in der Reaktionsmischung ein molares Verhältnis von $SiO_2/B_2O_3$ von 3 bis 300, bevorzugt von 10 bis 50 einstellt.

6. Verwendung der nach Anspruch 2 bis 5 hergestellten kristallinen Borosilikatzeolithe-ZBH als Katalysatoren für die Herstellung von Olefinen und/oder Aromaten aus Methanol und/oder Dimethylether.

**Revendications**

1. Zéolites borosilicate-ZBH de formule générale

$$M_{2/n}O:B_2O_3:x\ SiO_2:y\ H_2O$$

dans laquelle M représente au moins un cation de valence n, x s'élève à 10 et y se situe entre 0 et 80, suivant le diagramme de diffraction des rayons X simplifié suivant (1Å=0,1 nm).

| Ecartement des plans d (Å) | Intensité relative $I/I_o$ |
|---|---|
| 11.0240 ±0,15 | 93 |
| 9.8992 | 63 |
| 9.6825 | 20 |
| 3.8212 ±0,07 | 100 |
| 3.7908 | 73 |
| 3.7202 | 36 |
| 3,6901 | 49 |
| 3.6154 | 31 |
| 1.9975 | 9 |
| 1.9770 | 10 |

2. Procédé pour la préparation de zéolites borosilicate-ZBH cristallines du type pentasile selon la revendication 1 par cristallisation hydrothermale de dioxyde de silicium et d'acide borique en présence de sels alcalins, caractérisé en ce qu'on mène la cristallisation à des températures de 80 à 150°C en mélange alcoolique ou hydro-alcoolique.

3. Procédé selon la revendication 2, caractérisé en ce qu'on utilise, comme solvant pour la cristallisation, le 1,6-hexanediol et/ou le 1,4-butanediol.

4. Procédé selon la revendication 2 ou 3, caractérisé en ce que le mélange contient des sels alcalins dans des proportions de 1 à 3 mol par mol de $B_2O_3$.

5. Procédé selon l'une quelconque des revendications 2 à 4, caractérisé en ce qu'on règle dans le mélange réactionnel un rapport molaire $SiO_2/B_2O_3$ de 3 à 100, de préférence de 10 à 50.

6. Utilisation des zéolites borosilicate-ZBH cristallines préparées suivant l'une quelconque des revendications 2 à 5 comme catalyseurs pour la préparation d'oléfines et/ou de carbures aromatiques à partir de méthanol et/ou de diméthyléther.

**Claims**

1. A ZBH borosilicate zeolite of the general formula

$$M_{2/n}O:B_2O_3:x\ SiO_2:\ y\ H_2O,$$

where M is at least one cation with a valency n, x is 10 and y is from 0 to 80, and having the following simplified X-ray diffraction diagram:

| Interplanar spacing d (Å)*) | | Relative intensity $I/I_o$ |
|---|---|---|
| 11.0240 | ±0.15 | 93 |
| 9.8992 | | 63 |
| 9.6825 | | 20 |
| 3.8212 | ±0.07 | 100 |
| 3.7908 | | 73 |
| 3.7202 | | 36 |
| 3.6901 | | 49 |
| 3.6154 | | 31 |
| 1.9975 | | 9 |
| 1.9770 | | 10 |

*) 1Å=0.1 nm

2. A process for the preparation of a crystalline ZBH borosilicate zeolite of the Pentasil type as claimed in claim 1 by hydrothermal crystallization of silicon dioxide and boric acid in the presence of an alkali metal salt, wherein the crystallization is carried out at from 80 to 150°C in an alcoholic or aqueous-alcoholic medium.

3. A process as claimed in claim 2, wherein hexane-1,6-diol and/or butane-1,4-diol is used as solvent for the crystallization.

4. A process as claimed in claims 2 and 3, wherein the mixture contains from 1 to 3 moles of an alkali metal salt per mole of $B_2O_3$.

5. A process as claimed in claims 2 to 4, wherein the molar ratio of $SiO_2$ to $B_2O_3$ in the reaction mixture is from 3 to 300, preferably from 10 to 50.

6. Use of a crystalline ZBH borosilicate zeolite prepared according to claims 2 to 5, as a catalyst for the preparation of olefins and/or aromatics from methanol and/or dimethyl ether.